# EUROPEAN PATENT SPECIFICATION

(11) **EP 3 897 487 B1**
(45) Date of publication and mention of the grant of the patent: **02.12.2026**
(21) Application number: 19901251.9
(22) Date of filing: 28.10.2019
(51) Int. Cl.: A61F 9/02

(54) **ADJUSTABLE EYEWEAR STRAP**
VERSTELLBARES BRILLENBAND
SANGLE DE LUNETTES RÉGLABLE

(30) Priority: 21.12.2018 US 201862783340 P
(43) Date of publication of application: 27.10.2021
(73) Proprietor: OAKLEY, INC., Foothill Ranch, California 92610 (US)
(72) Inventor: BURNS, Cameron Scott, Foothill Ranch, California 92610 (US); GARFIAS, Nicolas Adolfo, Foothill Ranch, California 92610 (US); GODFREY, Chadd, Foothill Ranch, California 92610 (US); KIM, Joon H., Foothill Ranch, California 92610 (US); O'KEENE, Dugan, Foothill Ranch, California 92610 (US); WOOLLEY, David A., Foothill Ranch, California 92610 (US)
(74) Representative: Zacco Sweden AB
(86) International application number: PCT/US2019/058327
(87) International publication number: WO 2020/131219

(56) References cited:
- KR-A- 20150 125 270
- KR-A- 20150 125 270
- US-A- 2 066 838
- US-A- 2 066 838
- US-A- 4 974 956
- US-A- 4 974 956
- US-A1- 2014 189 944

## Description

### BACKGROUND

Eyewear, such as eyeglasses, sunglasses, goggles, and the like, must be removably attached to a user such that the eyewear is appropriately positioned in front of the user's eyes. Many different techniques have been used to attach eyewear to a user. For example, many different types of eyewear include two temples that extend along the sides of the user's head and hook over the user's ears. In many cases, attachment mechanisms, such as the above described temples, are either not usable, e.g., for use with a goggle, or do not provide sufficient retention of the eyewear during physical movement of the user. In these cases, the eyewear is often provided with a strap, such as a cord, fabric strip, or other flexible element that connects to the peripheral ends of the eyewear to form a loop. The loop can be passed around the user's head and can serve to secure the eyewear during physical movement of the user. Due to differences in head size and shape, it is often desirable to adjust the total length of the loop in order to optimize comfort and retention of the eyewear strap.

### BRIEF SUMMARY OF THE INVENTION

One aspect of the invention provides an eyewear strap adjustment lock to adjust the length of the strap. Adjustments can be made while the goggle is worn and the adjustment lock can be integrated into a strap and lens assembly. In an aspect, a user can tighten the eyewear strap while worn to achieve a more secure fit. The eyewear strap can include an internal cord and integrated lock. The lock can be a plate mounted to the strap connection. The elastic cord can be pulled to the desired fit and then locked to keep the strap at a desired length. The strap length can be extended by releasing the cord from the adjustment lock.

Another aspect of the invention provides an adjustable eyewear strap including a strap body having a first end configured to attach to a first portion of an eyewear and a second end configured to attach to a second portion of the eyewear. The adjustable eyewear strap can include an adjustment cord connected to the strap body and a cord lock configured to releasably engage the adjustment cord as it passes through the cord lock. The adjustment cord can be adjusted between at least a first position that results in a first length of the strap body, and a second position that results in a second length of the strap body, where the second length is less than the first length. Another aspect of the invention provides an eyewear assembly including a lens and an adjustable eyewear strap. The cord lock can be positioned on the lens.

An aspect of the invention provides an eyewear assembly including an eyewear and an adjustable strap attached to the eyewear. The adjustable strap can include a strap body, an adjustment cord connected to the strap body, and a cord lock disposed on the eyewear and configured to engage the adjustment cord in a locking position and release the adjustment cord in an adjusting position. The adjustment cord can be free to move through the cord lock when the adjustment cord is in the adjusting position and the adjustment cord cannot be free to move relative to the cord lock when the adjustment cord is in the locking position. The scope of protection of the invention is to be defined by the appended claims.

U.S. Patent No. 2,066,838 discloses an eye protective device having goggle cups formed to effectively cover the eyes and capable of being quickly adapted to any size head and being easily put on or off. It discloses an adjustment cord connected to the strap body, a cord lock mechanism configured to engage the adjustment cord at different positions to systematically shorten or lengthen the relaxed state of the strap assembly and a structural arrangement wherein a portion of the adjustment cord runs substantially parallel to the main strap body.

### BRIEF DESCRIPTION OF THE DRAWINGS/FIGURES

The accompanying drawings, which are incorporated herein and form part of the specification, illustrate aspects of the present invention and, together with the description, further serve to explain the principles of the invention and to enable a person skilled in the relevant art(s) to make and use the invention.
FIG. 1 is a perspective view of an eyewear strap according to various aspects of the invention.
FIG. 2 is a detail view of FIG. 1 according to various aspects of the invention.
FIG. 2a is a cross-sectional view of FIG. 2 along line 2A2A, according to various aspects of the invention.
FIG. 3 is a detail view of FIG. 1 according to various aspects of the invention.
FIG. 4a is a perspective view of an eyewear assembly including strap attached to an eyewear according to various aspects of the invention.
FIG. 4b is a perspective view of an eyewear assembly including strap attached to an eyewear according to various aspects of the invention.
FIG. 5a is a detail view of a locking mechanism according to various aspects of the invention.
FIG. 5b is a detail view of a locking mechanism according to various aspects of the invention.
FIG. 5c is a detail view of a locking mechanism according to various aspects of the invention.
FIG. 6 is a perspective view of a strap configuration according to various aspects of the invention.
FIG. 7 is a perspective view of a strap configuration according to various aspects of the invention.
FIG. 8 is a perspective view of an eyewear strap according to various aspects of the invention.
FIG. 9 is a perspective view of an eyewear assembly including an eyewear strap according to various aspects of the invention.
FIG. 10 is a perspective view of an eyewear assembly including an eyewear strap with according to various aspects of the invention.
FIG. 11 is a perspective view of an eyewear strap according to various aspects of the invention.
FIG. 12 is a section view of a cord locking mechanism according to aspects.
FIG. 13 is a perspective view of an eyewear strap according to various aspects of the invention.
FIG. 14 is a front view of an eyewear strap according to various aspects of the invention.
FIG. 15 is a front view of an eyewear strap according to various aspects of the invention.
FIG. 16 is a top view of an eyewear assembly according to various aspects of the invention.
FIG. 17 is a top view of an eyewear assembly according to various aspects of the invention.
FIG. 18 is a top view of an eyewear assembly according to various aspects of the invention.

### DETAILED DESCRIPTION OF THE INVENTION

The present invention(s) will now be described in detail with reference to aspects thereof as illustrated in the accompanying drawings. References to "one aspect," "an aspect," "an exemplary aspect," etc., indicate that the aspect described may include a particular feature, structure, or characteristic, but every aspect may not necessarily include the particular feature, structure, or characteristic. Moreover, such phrases are not necessarily referring to the same aspect. Further, when a particular feature, structure, or characteristic is described in connection with an aspect, it is submitted that it is within the knowledge of one skilled in the art to affect such feature, structure, or characteristic in connection with other aspects whether or not explicitly described.

Various mechanisms are used to retain eyewear in the proper position on the head of a user. The use of a strap, such as a cord, fabric strip, or other flexible element that forms a loop with the outer edges of the eyewear is a preferred method for retaining eyewear when the user is engaged in activities that involve physical movement, such as athletic activities. Differences in head size and shape make it desirable to adjust the total length of the eyewear strap to optimize the retention and comfort of the strap. One known mechanism includes one or more adjustment buckles (also known as a clip or adjuster) located at a midpoint of the strap. The adjustment buckles are configured to adjustably increase and decrease the length of the strap. Due to their location on the strap, the buckles are located on the back of the head of a user when the eyewear is being worn with the strap. When the user is wearing a head covering, such as a hat or helmet, the strap can be worn under the covering, which means that the covering must be removed to adjust the rear-mounted buckles described above. Alternatively, the strap can be worn over the head covering. Positioning the strap over the head covering can detrimentally affect the fit and retention of the eyewear and the fit of the head covering. Furthermore, the aforementioned buckles can render the strap aesthetically displeasing because the buckles are visible from the exterior of the strap.

According to the present disclosure, aspects can include an adjustable eyewear strap that has a strap body having a first end configured to attach to a first portion of an eyewear and a second end configured to attach to a second portion of the eyewear. An adjustment cord can be connected to the strap body and a cord lock can be configured to releasably engage the adjustment cord as it passes through the cord lock. A portion of the adjustment cord located before the adjustment cord enters the cord lock can be substantially parallel to the strap body. The adjustment cord can be adjusted between at least a first position that results in a first length of the strap body and a second position that results in a second length of the strap body. The second length can be less than the first length. Advantages of this and other aspects of the present disclosure include, for example, the ability to adjust the strap from alternative positions, such as near the temples of a user, and improved appearance.

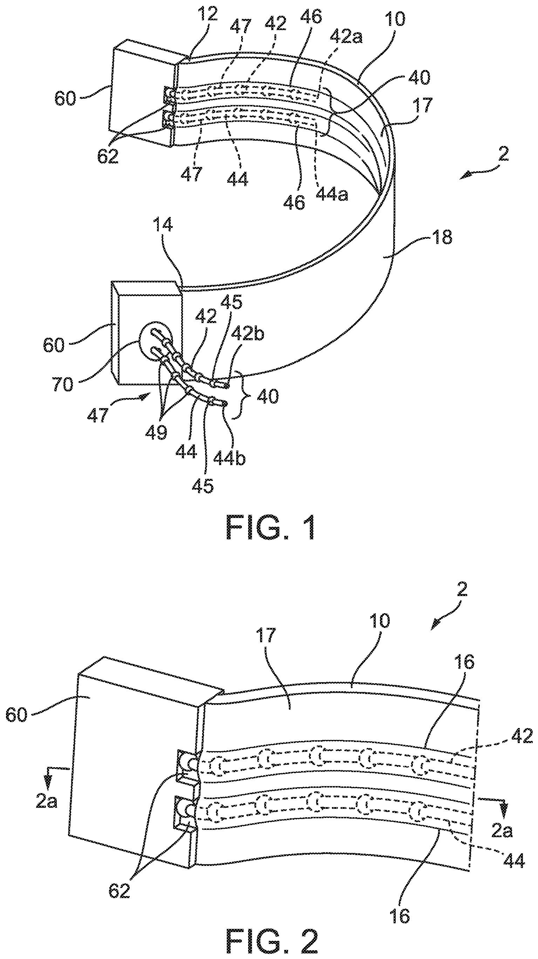

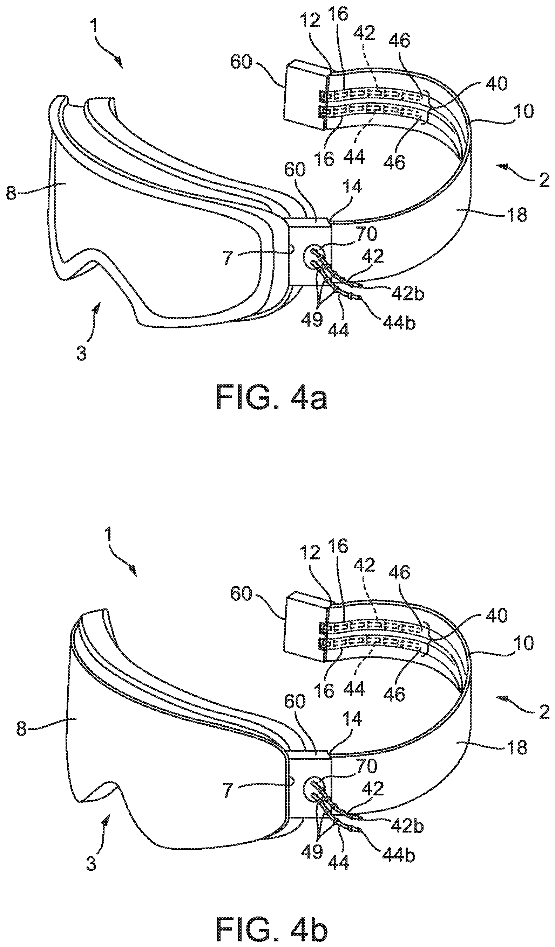

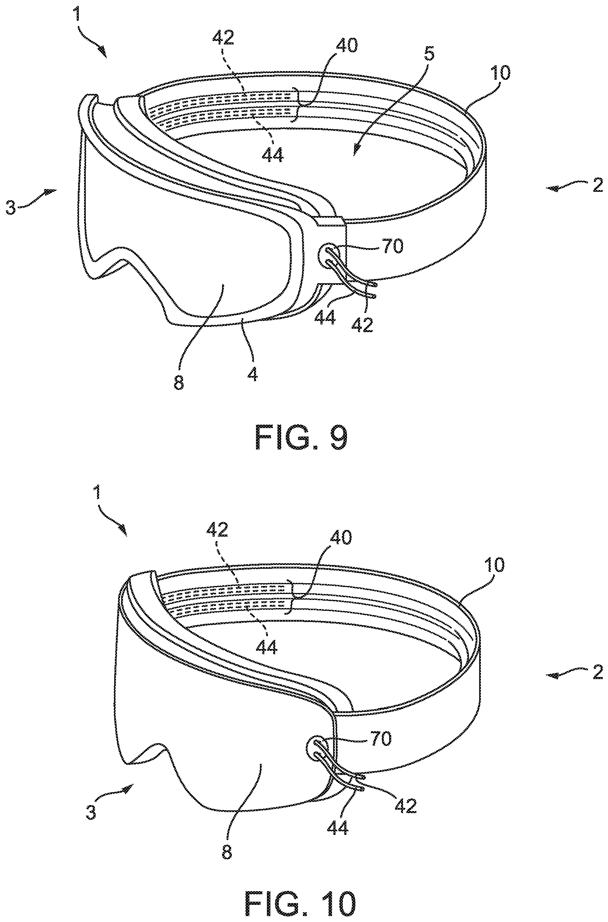

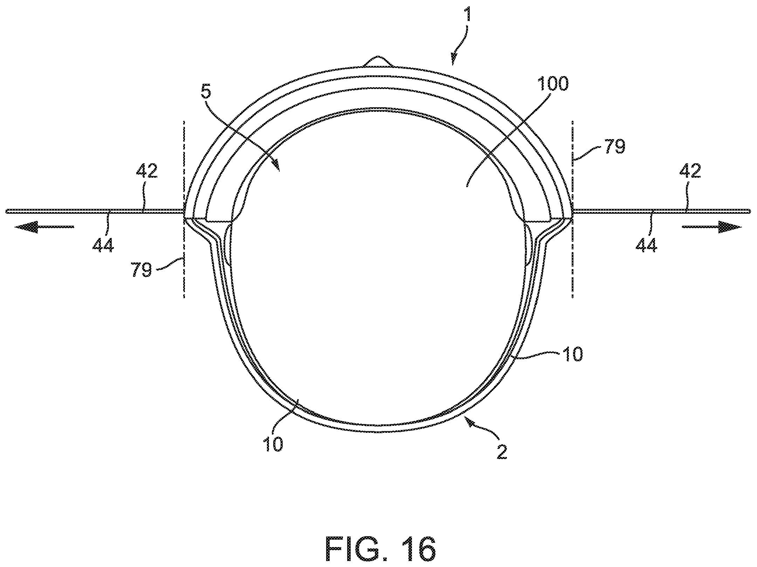

An aspect of an eyewear assembly 1 is shown in FIGS. 1-4b and can include an eyewear strap 2 and an eyewear 3. Eyewear 3 may include an eyewear frame 4 and a lens 8. Eyewear strap 2 can include a strap body 10 that includes a first end 12 and a second end 14. First end 12 and second end 14 can connect to a first portion 6 and second portion 7, respectively, of eyewear 3 (FIG. 4a-4b). Eyewear assembly 1 is shown as a goggle in FIGS. 4a-4b, but eyewear assembly 1 may be any appropriate type of eyewear, including eyeglasses, sunglasses, safety glasses, and the like. When connected to eyewear assembly 1, strap body 10 forms a loop 5 with eyewear assembly 1 (FIGS. 9-10). Loop 5 can encircle the head of a user and secure eyewear assembly 1 in the appropriate position in front of the user's eyes, as shown in FIGS. 16-18.

Strap body 10 can include a soft material that is able to conform to the shape of the user's head. In an aspect, strap body 10 can include an elastic material such as natural rubber, synthetic rubber, latex, nylon, stretch fabric (elastomerics), blended rubber with polyester, blended rubber with cotton, blended rubber with nylon or any combination thereof. In another aspect, strap body 10 can include a woven or nonwoven material, such as cotton, polyester, nylon, poly-vinyl chloride (PVC), rayon, microfiber, acrylic, acetate, wool, leather, vinyl, or any other suitable fabric that is able to withstand wear and tear and repeated stretching over time. Aspects of strap body 10 described here may also comprise elastic or non-elastic variations of the materials described above. For example, strap body 10 may include elasticized nylon or non-elasticized nylon.

Strap body 10 may be connected to or attached to a portion of eyewear 3. For example, as shown in FIGS. 4a-4b, first end 12 and second end 14 of strap body 10 may be attached to eyewear attachments 60, which are in turn releasably or permanently attached to first portion 6 and second portion 7 of eyewear 3. FIG. 4a shows aspects of eyewear assembly 1 where eyewear attachment 60 is removably attached to frame 4 of eyewear 3. In other aspects, strap body 10 may be connected or directly attached to an eyewear frame 4 of eyewear assembly 1 (FIG. 9). In another aspect, as shown in FIG. 4b, strap body 10 may be removably or permanently connected directly to a lens 8 of eyewear assembly 1 as shown in FIGS. 4b and 10, respectively. Strap body 10 may be directly attached to attachments 60, eyewear frame 4, or lens 8 using any appropriate method, including forming a woven loop, over molding around strap body 10, welding, or using adhesive.

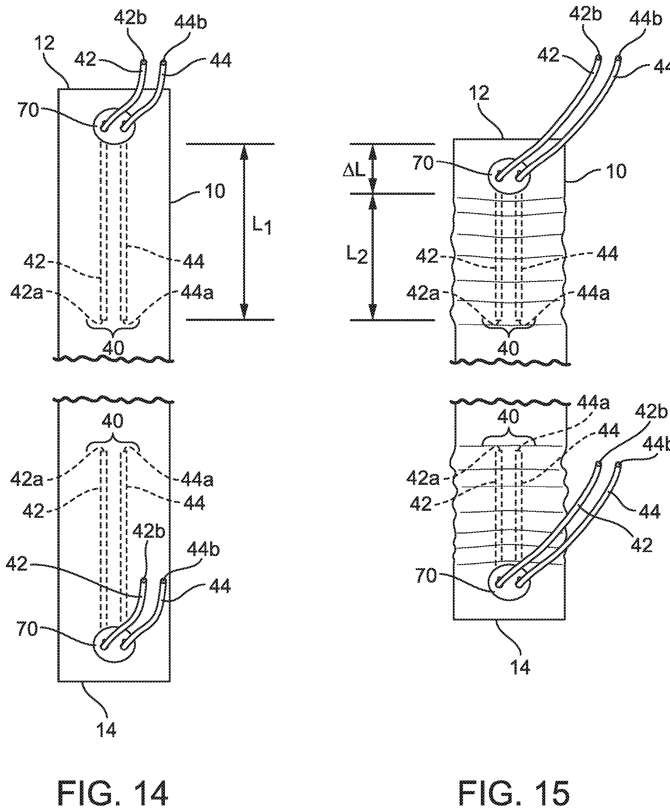

Eyewear strap 2 may be adapted to function with various head sizes. A typical small head size has a circumference of between 51 and 54 cm. A typical extra-large head size may have a circumference from 61 to 65 cm. Intermediate head sizes, that may be medium or large, fall in between these ranges. In aspects, eyewear strap 2 can have a length between first end 12 and second end 14 of between approximately 40 mm +/- 10 mm and approximately 100 mm +/- 10 mm. As shown in FIGS. 1-4, eyewear strap 2 can include an adjustment cord 40 attached to a portion of strap body 10. Adjustment cord 40 can operate in conjunction with a cord lock 70 to adjust the total length of eyewear strap 2 between first end 12 and second end 14. FIGS. 14 and 15 are front views of an aspect of eyewear strap 2 showing different adjusted lengths of eyewear strap 2. A total adjustable length L1 is the relaxed or unshortened length of an adjustment region of eyewear strap 2 that extends from the point of cord connection 46 closest to cord lock 70 to cord lock 70. An adjusted length L2 shows the same adjustment region of eyewear strap 2 after it has been shortened by tightening adjustment cord 40. Thus, L2 may range from approximately 0 mm to approximately length L1. ΔL is the difference between L1 and L2.

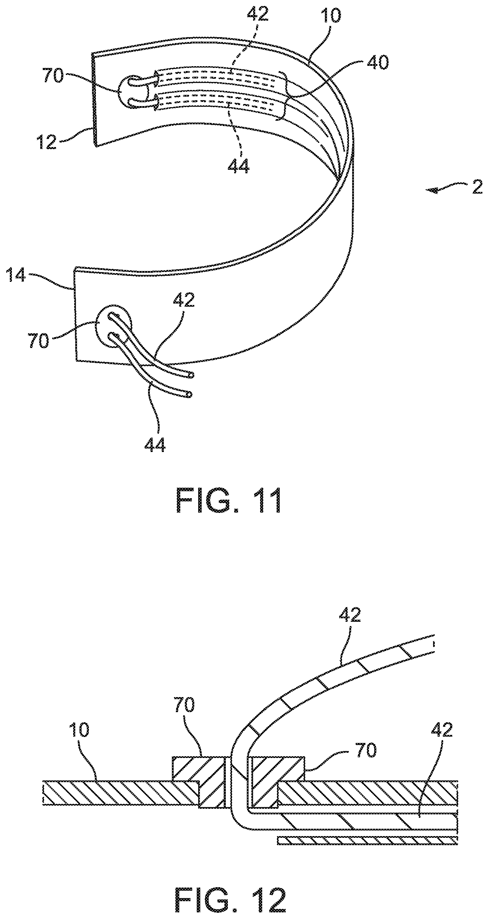

L1 and L2 quantify the adjustment region of eyewear strap 2 that can be extended or "bunched" as needed to affect the change in length of eyewear strap 2. For example, FIG. 14 shows eyewear strap 2 at a maximum length, or unadjusted state, and thus length L1 is shown. FIG. 15 shows the same view of eyewear strap 2, however eyewear strap 2 has been shortened through use of adjustment cord 40 and cord lock 70, and thus length L2 is shown. FIGS. 14 and 15 show aspects of eyewear strap 2 with at least one cord lock 70 and corresponding adjustment region, for example cord lock 70 at first end 12 of eyewear strap 2, as shown. It should be appreciated that aspects of eyewear strap 2 may have more than one cord lock 70 and associated adjustment regions, each of which has respective lengths L1, L2, and ΔL, for example, the second cord lock 70 and corresponding adjustment region at second end 14 of eyewear strap as shown in FIGS. 14 and 15. The lengths L1, L2, ΔL may be distributed between the multiple cord locks 70 and their respective adjustment regions as desired. For example, as shown in FIGS. 11, 14, and 15, there may be two cord locks 70, each of which is located near one of first end 12 and second end 14. In aspects, it may be desirable to adjust each adjustment region evenly, which means that each adjustment region would be associated with length values that are one half of the total length values for eyewear strap 2. In another aspect with a pair of cords 40, both or one of the pair of chords 40 can be associated with length values that are up to the full length of the values discussed below. Thus, in aspects of eyewear strap 2 with two cord locks 70, such as the one shown in FIG. 11, the adjustment regions associated with either cord lock 70 may be associated with one half of the total ΔL value after adjustment of eyewear strap 2.

In aspects, for example, L1 may range from approximately 0 mm to approximately 100 mm, such as from approximately 0 mm to approximately 80 mm, such as from approximately 0 mm to approximately 60 mm, such as from approximately 0 mm to approximately 50 mm, such as from approximately 10 mm to approximately 40 mm, such as from approximately 20 mm to approximately 30 mm. In an aspect, L1 can be approximately 27 mm +/- 10 mm. It should be appreciated that the specific length L1 of an aspect of eyewear strap 2 may be modified to any desirable length to provide a range of adjustment for eyewear strap 2, and thus the lengths L1 given above are exemplary in nature. The difference between L1 and L2, ΔL, varies depending on the specific configuration of eyewear strap 2, the head size of the user, and the desired fit, i.e. a tight fit versus a loose fit. For example, ΔL can allow adjustment from a small head size to an extra-large head size. In an aspect based on a head circumference of 54 cm, for example, L1 may range from approximately 20 mm to approximately 50 mm and L2 may range from approximately 0 mm to approximately 50 mm, resulting in a ΔL of between approximately 0 mm and approximately 50 mm. In another aspect based on a head circumference of 54 cm, where eyewear strap 2 features two cord locks 70, for example as shown in FIG. 11, each cord lock 70 may be associated with an L1 of approximately 27 mm and an L2 of approximately 17 mm. This results in a ΔL of approximately 10 mm for each of the two cord locks 70, and thus the total ΔL for eyewear strap 2 is 20 mm. Non-zero values of L2, which correspond to a less than fully shortened eyewear strap 2, will result in values of ΔL that are less than L1. In aspects, the adjustment region may not be configured to be completely shorted or collapsed. That results in a length L2 that has a lower bound greater than zero. In these aspects, the maximum ΔL possible would be less than the full length L1. In aspects associated with large or extra-large head sizes, there may be little or no adjustment of eyewear strap 2 required. When eyewear strap 2 is adjusted to the shortest possible length in any given aspect, with adjustment cord 40 being pulled through cord lock 70 to the fullest extent possible, L2 will equal 0 mm and ΔL will be equal to L1. In some aspects of eyewear strap 2 that comprises an elastic material, L2 may be greater than L1. For example, in an aspect based on a head size of 63 cm, L1 may be approximately 27 mm and L2 may be approximately 41 mm, which results in a ΔL of approximately -14 mm. This is possible because these aspects of eyewear strap 2 can stretch to accommodate larger head sizes.

It should be appreciated that aspects of eyewear strap 2 may be adjusted to any number of positions between and beyond L1, and thus the specific values discussed here are exemplary in nature and are not limiting.

In an aspect, adjustment cord 40 can be positioned on an interior side 17 of strap body 10 such that adjustment cord 40 would be positioned adjacent a wearer's head. In another aspect, adjustment cord 40 can be positioned on an exterior side 18 of strap body 10 such that adjustment cord 40 would be separated from a wearer's head by strap body 10. In a further aspect, adjustment cord 40 can be positioned on both interior side 17 and exterior side 18 of strap body 12 along one or more portions of strap body 10.

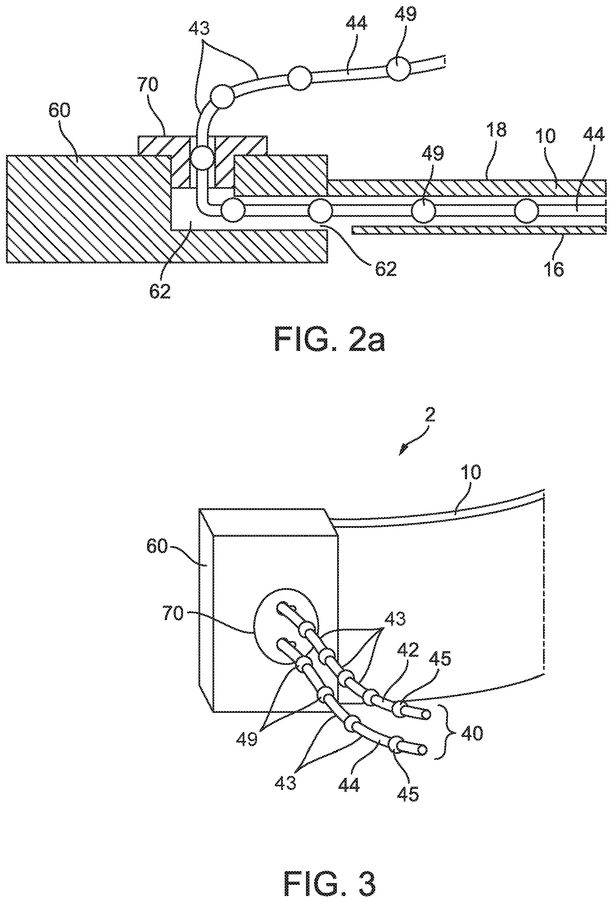

In an aspect, adjustment cord 40 may include one or more cord elements. For example, as shown in FIGS. 1 and 2, adjustment cord 40 may comprise a first cord 42 and a second cord 44. In an aspect, first cord 42 and second cord 44 can be positioned substantially parallel to each other, for example, along a portion of strap body 10. First cord 42 and second cord 44 may be made from any flexible elastic or non-elastic material. In aspects, cords 42 and 44 are made from an elastic cord, commonly called shock cord or bungee cord. In another aspect, cords 42 and 44 can be made from a flat, flexible material similar to that of aspects of strap body 10 as shown in FIG. 1, or any other appropriate flexible material. In an aspect, first cord 42 and/or second cord 44 can include an elastic material such as natural rubber, synthetic rubber, latex, nylon, stretch fabric (elastomerics), blended rubber with polyester, blended rubber with cotton, blended rubber with nylon or any combination thereof. In another aspect, first cord 42 and/or second cord 44 can include a woven or nonwoven material, such as cotton, polyester, nylon, poly-vinyl chloride (PVC), rayon, microfiber, acrylic, acetate, wool, leather, vinyl, or any other suitable fabric that is able to withstand wear and tear and repeated stretching over time.

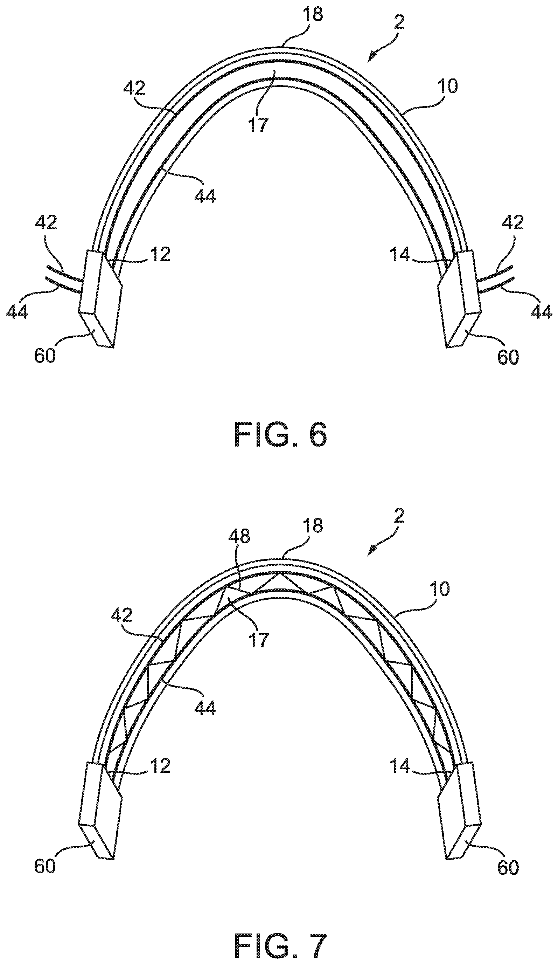

As shown in FIGS. 6-7, adjustment cord 40 may extend continuously between first end 12 and second end 14 of strap body 10. FIG. 6 shows a perspective view of the interior of aspects of eyewear strap 2 that has such a configuration of adjustment cord 40. In one aspect, adjustment cord 40 may comprise interlacing 48 that extends between first cord 42 and second cord 44, as shown in FIG. 7. Interlacing 48 may be configured to enhance the comfort of adjustment cord 40 against the head of a user when adjustment cord 40 is tightened.

A portion of adjustment cord 40 may be fixed to strap body 10. For example, as shown in FIGS. 1 and 4, adjustment cord 40 may be fixed to strap body 10 at cord connection 46. Cord connection 46 may be an area, for example, where the distal ends 42a and 44a of cords 42 and/or 44 meet strap body 10. In another aspect, cord connection 46 may be a region extending a small distance along cord 42 and/or 44 and a portion of strap body 10 near ends 42a and 44a. Cord connection 46 is configured to fix adjustment cord 40 to strap body 10 such that the portion of adjustment cord 40 that coincides with cord connection 46 is not capable of movement relative to strap body 10. In an aspect, cord connection 46 can be located at an end of adjustment cord 40 that is located opposite first end 12 and second end 14 of strap body 10. Adjustment cord 40 may be fixed to strap body 10 at cord connection 46 through any appropriate method or combination of methods, including weaving, sewing, adhesives, or plastic welding. In an aspect, cord connection 46 can be a semi-permanent connection to facilitate replacement of adjustment cord 40.

In an aspect, adjustment cord 40 may include scale 49, as shown in FIGS. 1-3. Scale 49 may include evenly-spaced marks or indications, similar to those on a ruler, that allow a user to measure the portion of adjustment cord 40 that is exterior to eyewear strap 2. This enables a user to accurately make repetitive adjustments (i.e. adjusting the total strap length the same amount every time). In aspects, the marks or indications of scale 49 may be a different color than the remainder of adjustment cord 40 to provide a visual indication of the length of adjustment cord 40. In aspects, the marks or indications of scale 49 may be physically larger than an adjacent portion of adjustment cord 40, as shown in FIGS. 1-3. This allows a user to receive tactile feedback as to the amount of adjustment cord 40 that has been withdrawn from cord lock 70. For example, in addition to physical touching the different size indicators or markers on adjustment cord 40, the indicators or markers may be sized to rub or bump against cord lock 70 when adjustment cord 40 is being adjusted. This may give a user another form of tactile feedback when adjustment cord 40 is being adjusted. The tactile aspects of scale 49 may also be a different color than the remainder of adjustment cord 40 to enhance visual feedback. As discussed below, the tactile aspects of adjustment cord 40 may also enhance the security of the adjusting mechanism of eyewear 1.

In another aspect, the indications on scale 49 can be un-evenly spaced to account for both macro and micro adjustments.

In an aspect, a portion 45 of adjustment cord 40 can be distal to cord connection 46 and can be movable with respect to strap body 10. In an aspect, the distal ends of cords 42 and 44 can converge at portion 45 such that cords 42 and 44 move in unison. In an aspect, a portion 47 of adjustment cord 40 can be connected to strap body 10 in a slidable manner. For example, as shown in FIG. 2, which is a detail view of the upper portion of FIG.1, adjustment cord 40 may be positioned in an adjustment cord housing 16 that is disposed on strap body 10. Adjustment cord housing 16 can include one or more channels that surrounds adjustment cord 40. In another aspect, strap body 10 can include an adjustment cord housing 16 to surround first adjustment cord 42 and another adjustment cord housing 16 to surround second adjustment cord 44. For example, FIG. 4a shows individual housings 16 for cords 42 and 44. In another aspect, a single housing 16 may accommodate both cords 42 and 44-i.e. the two distinct housings 16 shown in FIG. 4a may instead be one continuous housing 16. Adjustment cord housing 16 can be made of a material similar to that of strap body 10. In another aspect, adjustment cord housing 16 can be sewn, woven, or otherwise fixed to strap body 10. In an aspect, one or more of first adjustment cord 42 and second adjustment cord 44 can pass through one or more adjustment cord housings 16. Thus first adjustment cord 42 and/or second adjustment cord 44 can be retained next to strap body 10 while portions of 42 and/or 44 remain unconstrained and free to slide back and forth along a portion of strap body 10. It should be appreciated that it is within the scope of this disclosure that the various aspects of housing 16-with single or multiple distinct compartments-can be used with any of the aspects of adjustment cord 40.

In an aspect, strap body 10 may be formed from two or more layers of flexible material. Adjustment cord 40 may be positioned between the two layers of strap body 10 such that portion 47 of adjustment cord 40 is free to slide relative to strap body 10. Thus, adjustment cord housing 16 can be formed by multiple layers of strap body 10. Other aspects of adjustment cord housing 16 may include discrete loops or clips fixed to strap body 10 that slidably retain adjustment cord 40. Adjustment cord 40 can move relative to strap body 10 such that a length of adjustment cord 40 between cord connection 46 and cord lock 70 is modified. Because adjustment cord 40 is attached to strap body 10 at cord connection 46, movement of adjustment cord 40 in turn alters the length of strap body 10 between ends 12 and 14. For example, the length of adjustment cord 40 between cord connection 46 and cord lock 70 can increase or decrease to alter the length of strap body between ends 12 and 14. In an aspect, strap body 10 can gather and release adjustment cord 40 as necessary to account for the differing lengths of adjustment cord 40. Accordingly, in some aspects where a desired length adjustment range, ΔL, is large, the total length of adjustment cord 40 that extends from strap body 10 can be relatively greater than the portion of adjustment cord 40 that remains within adjustment cord housing 16.

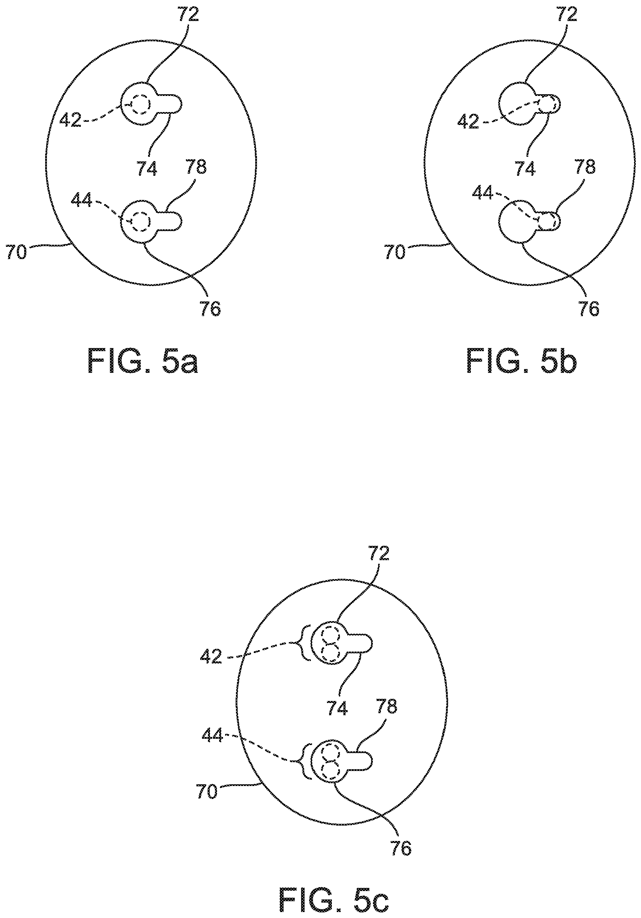

As shown in FIGS. 1-5, eyewear strap 2 can utilize cord lock 70. Cord lock 70 can releasably retain adjustment cord 40 to prevent movement of adjustment cord 40 with respect to strap body 10. As shown in FIGS. 5a-5c, cord lock 70 can include a first cord passage 72 and a first cord lock passage 74. Adjustment cord 40 can pass through cord lock 70 through either of cord passage 72 or cord lock passage 74. First cord passage 72 and first cord lock passage 74 can be interconnected such that adjustment cord 40 may move, translate, or shift between either first cord passage 72 and first cord lock passage 74. In an aspect, the cross-sectional area of first cord passage 72 can be sized to allow adjustment cord 40 to freely pass through cord lock 70. The cross-sectional area of first cord lock passage 74 can be sized to engage adjustment cord 40 to prevent movement of adjustment cord 40 relative to cord lock 70. As shown in FIG. 5a, first cord passage 72 can have a cross-sectional area that is larger than a cross sectional area of adjustment cord 40. In an aspect as shown in FIG. 5b, first cord lock passage 74 can have a cross-sectional area that is smaller than a cross sectional area of adjustment cord 40. In aspects, first cord passage 72 and first cord lock passage 74 may be sized to function with two or more cord elements of adjustment cord 40 , as shown in FIG. 5c. In these aspects, first cord passage 72 is sized to allow the two or more cords through cord lock 70 freely, while first cord lock passage 74 is sized to releasably engage the two or more cords simultaneously.

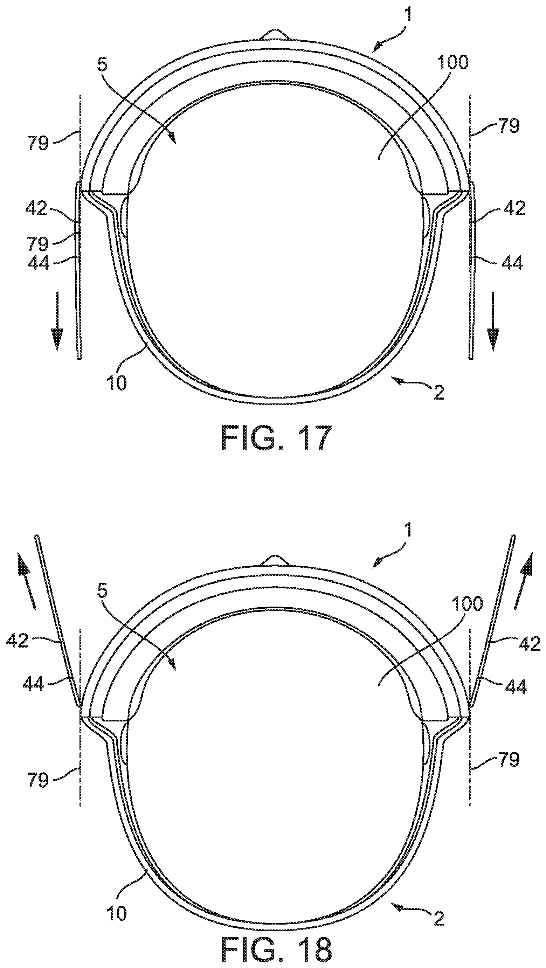

The interconnection of cord passage 72 and cord lock passage 74 can enable a user to adjust the length of adjustment cord 40 through first cord passage 72, and then secure the desired length of strap body 10 by moving, translating, or shifting adjustment cord 40 into first cord lock passage 74. The orientation of cord lock 70 may vary depending on the desired direction of force on adjustment cord 40 needed to lock and unlock adjustment cord 40 in cord lock 70. As shown in FIGS. 5a-5C adjustment cord 40 can be locked in place with respect to cord lock 70 when adjustment cord 40 is moved from left to right in the figure. FIGS. 16-18 show an example of the direction of pull on ends 42b and 44b of cords 42 and 44 needed to adjust, lock, and unlock adjustment cord 40 respectively. In an aspect shown in FIGS. 16-18, a top-down view of eyewear assembly 1 on a head 100 of a wearer is shown. Arrows corresponding to adjustment cord 40 show a direction of pull in each of the figures. In FIG. 16, a user can pull adjustment cord 40 outward, in a direction substantially perpendicular to a plane 79 that is tangent to the exterior of cord lock 70, from strap body 10 to tighten strap body 10 against the wearer's head 100. In an aspect as shown in FIG. 17, a user can move adjustment cord 40 backward toward the rear of the wearer's head 100, in a direction substantially parallel to plane 79, to lock adjustment cord 40 in first cord lock passage 74. And as shown in FIG. 18, a user can move adjustment cord 40 toward the front of the wearer's head 100 to move adjustment cord 40 into first cord passage 72 to unlock adjustment cord 40 from first cord lock passage 74. The orientation of cord lock 70 shown in FIGS. 5A-5C and illustrated in user in FIGS. 16-18 is exemplary. Various other orientations may be employed if desired. For example, in another aspect, cord lock 70 may also be oriented to position first cord lock passage 74 above first cord passage 72 or below first cord passage 72. In another aspect, cord lock 70 may also be oriented to position first cord lock passage 74 to the left of first cord passage 72, or to the right of first cord passage 72. That is to say, the narrower first cord lock passage 74 may be oriented in any direction desirable. This allows cord lock 70 to be configured such that adjustment cord 40 locks in first cord lock passage 74 when adjustment cord 40 is pulled in the desired direction- whether that is upwards, downwards, to the left, to the right, or any direction in between.

As shown in FIGS 5a-5C, cord lock 70 may include a second cord passage 76 and a second cord lock passage 78 that are substantially identical to first cord passage 72 and first cord lock passage 74. These aspects of cord lock 70 are designed for use with aspects of adjustment cord 40 that include multiple cords, such as first adjustment cord 42 and second adjustment cord 44 as shown in FIGS. 1-4b. In another aspect, cord lock 70 may include additional sets of cord and cord lock passages to accommodate additional elements of adjustment cord 40. Furthermore, the cross-sections of first cord passage 72 and first cord lock passage 74 may be modified to any appropriate shape based on the cross section of the cords of adjustment cord 40.

Aspects of adjustment cord 40 with scale 49 may also be configured to function with cord lock 70. For example, aspects of adjustment cord 40 with scale 49 that includes indications or markers that are physically larger may provide adjustment and locking of adjustment cord 40 in cord lock 70. This is because the locking passages of cord lock 70 (e.g. first cord lock passage 74 and second cord lack passage 78) may be have a smaller cross sectional area than that of the tactile elements of scale 49. This allows a user to repetitively select a specific adjustment length and securely lock it in place in cord lock 70 with greater accuracy. FIG. 2A shows an aspect of adjustment cord 40 with scale 49 that is physically larger than narrow portions 43 of adjustment cord 40 passing through cord lock 70. For example, by threading narrow portion 43 of cords 42 and 44 located between scale 49 into respective cord lock passages 74 and 78, a user may adjust the resulting length of cords 42 and 44 with greater accuracy.

Aspects of cord lock 70 may be fixed to, or incorporated in, various components of eyewear assembly 1, including, for example, lens 8, eyewear frame 4, eyewear attachment 60, and/or strap body 10.

As shown in FIGS. 1-4b and 8, cord lock 70 can be incorporated into eyewear attachments 60 that are connected to strap body 10, as discussed above. For example, adjustment cord 40 including one or more of first cord 42 and/or second cord 44 can be positioned on one or both sides (interior side 17 or exterior side 18) of strap body 10. Eyewear attachment 60 can include a cord channel 62 to allow adjustment cord 40 to pass through eyewear attachment 60 to first and second cord passages 72 and 76 and/or first and second cord lock passages 74 and 78 of cord lock 70. As shown in FIGS. 2-3, adjustment cord 40 can pass through cord channel 62 and exit the exterior of eyewear attachment 60 through cord lock 70. FIG. 2a shows a cross-section view of aspects of eyewear attachment 60. Adjustment cord 40 is shown passing through cord channel 62 and exiting out of cord lock 70. Note that the cross section of FIG. 2a is taken such that only cord passage 72 (or second cord passage 76) is visible. In an aspect, as shown in FIG. 2a, adjustment cord 40 can turn from the entrance to cord channel 62 to cord lock 70. For example, an exit path of adjustment cord 40 can make a substantially ninety degree turn as compared to an entry path of adjustment cord 40 prior to entry into cord channel 62 to cord lock 70. A user may grasp the one or more of ends 42b and 44b of adjustment cord 40 and alternatively pull or release adjustment cord 40 with respect to first and/or second cord passages 72 and 76 to first and/or second cord lock passages 74 and 78 to adjust the total length of strap body 10.

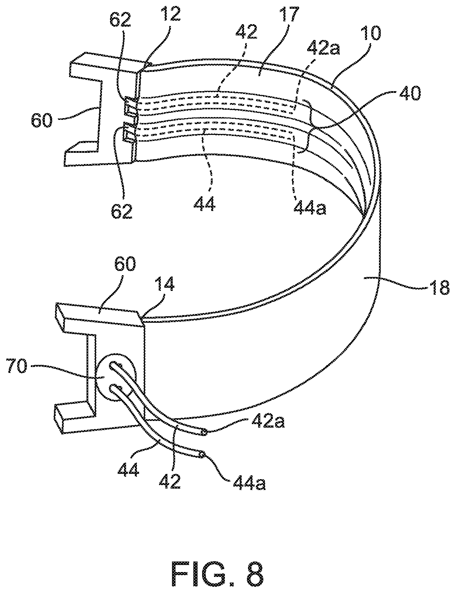

FIG. 8 shows another aspect of eyewear attachment 60 that otherwise functions identically to aspects discussed in FIGs with regards to cord lock 70 and adjustment cord 40. Such eyewear attachment 60 can resemble a conventional goggle strap attachment, or "outrigger", and attached to a goggle frame in a similar manner as conventional goggle outriggers, e.g., eyewear attachment 60, and can be pivotally or fixedly attached to eyewear frame 4 of eyewear 3. Exemplary, nonlimiting "outrigger" configurations which can be employed in the embodiment of FIG. 8 are discussed in greater detail in U.S. Patent No. 6,047,410, the disclosure of which is incorporated herein by reference.

In another aspect, cord lock 70 may be incorporated directly into a portion of eyewear assembly 1. For example, as shown in FIG. 9, cord lock 70 may be incorporated into eyewear frame 4. In another aspect shown in FIG. 10, cord lock 70 may be incorporated into lens 8. These aspects function similarly to the aspects of cord lock 70 incorporated into eyewear attachment 60 described above. The features and functionality of cord lock 70 discussed above in paragraphs 43-48 applies equally to these aspects.

In a further aspect, cord lock 70 may be positioned on strap body 10. As shown in FIG. 11, cord lock 70 may be attached to a portion of strap body 10. In an aspect, cord lock 70 can be positioned on an exterior 18 of strap body 10, opposite of cord connection 46 that is positioned on an interior surface of strap body 10. However, as discussed above, other aspects may alter the respective positioning of cord lock 70 and/or cord connection 46 and adjustment cord 40. In aspects, cord lock 70 may be positioned near ends 12 and 14 of strap body 10, as shown in FIG. 11. However, cord lock 70 may be positioned anywhere along strap body 10, as desired. For example, a single cord lock 70 may be positioned at the midpoint of strap body 10. The aspect of cord lock 70 shown in FIG. 5C is particularly suited for placement at the midpoint of strap body 10 because it is adapted to function with aspects of adjustment cord 40 that consist of doubled cord elements in each passage.

Figure 12 is a cross section of cord lock 70 according to aspects such as the one shown in FIG. 11. Note that the cross section of FIG. 12 is taken such that only cord passage 72 (or second cord passage 76) is visible. As shown in FIG. 12, placement of cord lock 70 on strap body can allow for passage of first cord 42 from a first side of strap body 10 to a second side of strap body 10. These aspects are otherwise functionally similar to aspects of cord lock 70 incorporated into eyewear attachment 60 or eyewear assembly 1 described above. The features and functionality of cord lock 70 discussed above in paragraphs 43-48 apply equally to these aspects.

Aspects of cord lock 70 may be constructed from any appropriate material. For example, cord lock 70 may be a rigid material, such as a metal, plastic, or composite material. Cord lock 70 may be shaped in any desired manner, such as an oval (as shown in FIGS. 5a-5c), a rectangle, or a circle. Cord lock 70 may be planar (i.e. substantially flat) or may be curved to match a corresponding mounting surface, for example.

In one aspect, cord lock 70 can be a separate component that connects to a portion of strap body 10, eyewear attachment 60, eyewear frame 4, or lens 8. For example, cord lock 70 may be fixed to eyewear attachment 60 or other components of eyewear assembly 1 using an adhesive. In another aspect, cord lock 70 can be detachably attached to eyewear attachment 60 or other components of eyewear assembly 1 using a press fit or snap fit connection. In another aspect, cord lock 70 can attach to strap body 10 using any appropriate method, including, for example, weaving, adhesives, welding, over molding, and other similar techniques.

In an aspect, cord lock 70 can be integrally formed into a portion of eyewear assembly 1. For example, cord lock 70 can be integrally formed into an eyewear attachment 60 during manufacture of eyewear attachment 60, such as by a molding process. In another aspect, cord lock 70 may be integrally formed into eyewear assembly 1. For example, cord lock 70 can be integrally formed into eyewear frame 4 or lens 8.

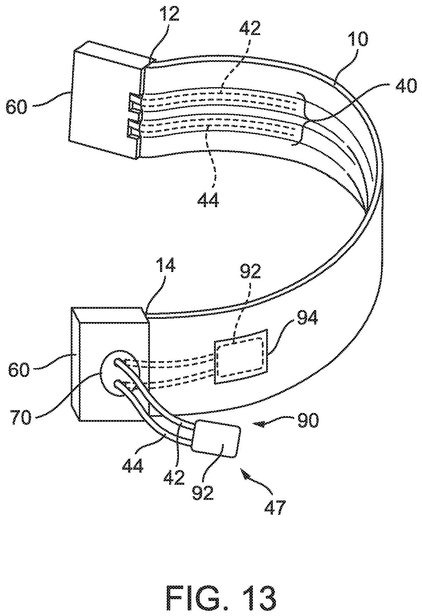

As shown in FIG. 13, aspects of the present disclosure may include a retainer 90 configured to releasably retain an excess portion of adjustment cord 40 that extends from cord lock 70. In an aspect, retainer 90 can be magnetic and can include an adjustment cord magnet 92 disposed on distal end 47 of adjustment cord 40. A base 94 can be fixed to at least a portion of eyewear assembly 1, for example, lens 8, eyewear frame 4, or body strap 10. Adjustment cord magnet 92 and base 94 can be configured to possess sufficient magnetic attraction to secure retainer 90 to base 94 and retain the excess length of adjustment cord 40 in a releasable manner. Base 94 may be positioned in a location that is convenient for releasable storage of the excess adjustment cord 40. For example, base 94 may be positioned immediately to the rear, with respect to a user, of cord lock 70 on strap body 10. In an aspect, base 94 may be positioned on a corresponding piece of headwear of a user, for example a hat or helmet. In another aspect, base 94 may be positioned at the same location as cord connection 46. In aspects, base 94 may be positioned at the same point of strap body 10 as cord connection 46, but it may be disposed on exterior surface 18 of strap body 10 if cord connection 46 is located on interior surface 17. In another aspect, retainer 90 and base 94 can include a hook and loop connection, a snap fit connection, or other means to detachably attach retainer 90 to base 94.

In one aspect, any of the above described aspects can be used in conjunction with a conventional adjustment buckle. Such a combination may provide several advantages over the adjustment buckle known in the art, for example, adding fine/micro adjustability and improving ease of adjustment when the strap is worn under a head covering. For example, the aspect shown in FIG. 1, wherein cord lock 70 is on eyewear attachment 60, may be combined with an adjustment buckle or clip located at the midpoint of strap body 10 to improve ease of strap adjustment while retaining the traditional adjustment buckle/clip. In another aspect, the eyewear assembly of FIG. 10, which shows cord lock 70 integrated into lens 8, may also include an adjustment clip or buckle located at the midpoint of strap body 10.

In another aspect, any of the above described aspects can be used in place of an adjustment buckle to improve fine tuning of eyewear strap 2 and improve ease of adjustability when eyewear strap 2 is worn under a head covering.

A method of using aspects of the present disclosure begins with an initial fitting step. Adjustment cord 40 can be disengaged from cord lock 70 such that adjustment cord 40 is moved into the first cord passage 72 (and second cord passage 76 as necessary). Accordingly, adjustment cord 40 can pass through cord lock 70 to extend the length of body strap 10 between ends 12 and 14 (FIG. 16). Eyewear assembly 1 can then be placed around the head 100 of a user in a donning step. In an adjustment step, the ends of adjustment cord 40 are pulled through first cord passage 72 (and second cord passage 76 as necessary) until a desired tension of body strap 10 is reached (FIG. 16). Adjustment cord 40 can then be pulled rearward such that adjustment cord 40 moves into first cord lock passage 74 (and second cord lock passage 78 as necessary) in a securing step to fix the desired length of body strap 10 (FIG. 17). In a storage step, the excess amount of adjustment cord 40 may be secured to magnetic base 94 by moving adjustment cord magnet 92 into proximity with magnetic base 94.

The foregoing description of the specific aspects will so fully reveal the general nature of the invention that others can, by applying knowledge within the skill of the art, readily modify and/or adapt for various applications such specific aspects, without undue experimentation, without departing from the general concept of the present invention.

## Claims

1. An adjustable eyewear strap (2), comprising:
a strap body (10) having a first end (12) configured to attach to a first portion (6) of an eyewear and a second end (14) configured to attach to a second portion (7) of the eyewear;
an adjustment cord (40) connected to the strap body; and
a cord lock (70) configured to releasably engage the adjustment cord as it passes through the cord lock,
wherein a portion of the adjustment cord located before the adjustment cord enters the cord lock is substantially parallel to the strap body,
wherein the adjustment cord can be adjusted between at least:
a first position that results in a first length of the strap body, and
a second position that results in a second length of the strap body, wherein the second length is less than the first length,
a second adjustment cord (44) connected to the strap body; and
a second cord lock configured to releasably engage the second adjustment cord as it passes through the second cord lock, comprising:
a second cord passage (72, 76) configured to allow the second adjustment cord to pass through the second cord lock; and
a second cord lock passage (74, 78) configured to releasably engage the adjustment cord to prevent movement of the adjustment cord through the second cord lock,
wherein the second cord lock passage is interconnected with the second cord passage such that the second adjustment cord is configured to move between the second cord passage and the second cord lock passage, and
wherein the second adjustment cord may be adjusted between at least:
a first position that results in a first length of the strap body, and
a second position that results in a second length of the strap body, wherein the second length is less than the first length.

2. The adjustable eyewear strap of claim 1, wherein the cord lock comprises:
a cord passage configured to allow the adjustment cord to pass through the cord lock; and
a cord lock passage configured to releasably engage the adjustment cord to prevent movement of the adjustment cord through the cord lock,
wherein the cord lock passage is interconnected with the cord passage such that the adjustment cord is configured to move between the cord passage and the cord lock passage, and
wherein the adjustment cords comprises an elastic material such that the adjustment cord returns to an initial length after being stretched beyond the initial length.

3. The adjustable eyewear strap of claim 1, wherein the cord lock is configured to turn the adjustment cord as it passes through the cord lock such that an adjustment cord exit path is not parallel to an entry path of the adjustment cord prior to entry of the adjustment cord into the cord lock.

4. The adjustable eyewear strap of claim 3, wherein the exit path is perpendicular to the entry path.

5. The adjustable eyewear strap of claim 1,
wherein a first portion of the adjustment cord is connected to the strap body at a first end of the adjustment cord such that the first end is not capable of movement relative to the strap body, and
wherein a second portion of the adjustment cord disposed between the first portion and the cord lock is not fixed to the strap body, such that the length of the second portion varies as the adjustment cord is adjusted through the cord lock.

6. The adjustable eyewear strap of claim 1, wherein the cord lock is attached to the strap body.

7. The adjustable eyewear strap of claim 1, wherein the cord lock is configured to pass the adjustment cord from an interior side of the strap body to an exterior side of the strap body.

8. The adjustable eyewear strap of claim 7, wherein an end portion of the adjustment cord that is disposed on the exterior side (18) comprises a grasping member configured to be grasped by a user.

9. The adjustable eyewear strap of claim 1, further comprising:
a retainer (90) positioned at a first end of the adjustment cord; and
a receiver attached to the strap body,
wherein the retainer is configured to releasably attach to the receiver.

10. An eyewear assembly, comprising:
an eyewear (1); and
the adjustable eyewear strap of claim 1.

11. The eyewear assembly of claim 10,
wherein the eyewear comprises a lens (8), and
the cord lock is positioned on the lens.

12. The eyewear assembly of claim 10, wherein a first end of the eyewear strap is attached to a first portion of the eyewear and a second end of the eyewear strap is attached to a second portion of the eyewear such that the eyewear and the eyewear strap form a loop configured to encircle a head of a user.

13. The adjustable eyewear strap of claim 1, wherein the adjustment cord comprises a scale (49) configured to indicate a length of the adjustment cord.

14. The eyewear assembly of claim 10, further comprising an eyewear frame (4),
wherein the cord lock is integral to the eyewear frame.

15. The adjustable eyewear strap of claim 1, wherein a length of the adjustment cord is adjusted by pulling the cord in a direction perpendicular to a plane (79) tangent to an exterior surface of the cord lock, and
wherein the adjustment cord is secured in the cord lock by pulling the cord in a direction parallel to the exterior plane.

16. The adjustable eyewear strap of claim 1, wherein:
the first position corresponds to a low-tension state in the adjustable strap, and
the second position corresponds to a high-tension state in the adjustable strap.

## Patentansprüche

1. Verstellbares Brillenband (2), umfassend:
einen Bandkörper (10), der ein erstes Ende (12), das dazu ausgelegt ist, an einem ersten Abschnitt (6) einer Brille angebracht zu werden, und ein zweites Ende (14), das dazu ausgelegt ist, an einem zweiten Abschnitt (7) der Brille angebracht zu werden, aufweist;
eine Verstellschnur (40), die mit dem Bandkörper verbunden ist; und
eine Schnurverriegelung (70), die dazu ausgelegt ist, die Verstellschnur lösbar in Eingriff zu nehmen, wenn sie durch die Schnurverriegelung verläuft,
wobei, bevor die Verstellschnur in die Schnurverriegelung eintritt, sich ein Abschnitt der Verstellschnur im Wesentlichen parallel zu dem Bandkörper befindet,
wobei die Verstellschnur zwischen mindestens Folgendem verstellt werden kann:
einer ersten Position, die zu einer ersten Länge des Bandkörpers führt, und
einer zweiten Position, die zu einer zweiten Länge des Bandkörpers führt, wobei die zweite Länge kürzer als die erste Länge ist,
eine zweite Verstellschnur (44), die mit dem Bandkörper verbunden ist; und
eine zweite Schnurverriegelung, die dazu ausgelegt ist, die zweite Verstellschnur lösbar in Eingriff zu nehmen, wenn sie durch die zweite Schnurverriegelung verläuft, umfassend:
einen zweiten Schnurdurchlass (72, 76), der dazu ausgelegt ist, der zweiten Verstellschnur zu ermöglichen, durch die zweite Schnurverriegelung zu verlaufen; und
einen zweiten Schnurverriegelungsdurchlass (74, 78), der dazu ausgelegt ist, die Verstellschnur lösbar in Eingriff zu nehmen, um eine Bewegung der Verstellschnur durch die zweite Schnurverriegelung zu verhindern,
wobei der zweite Schnurverriegelungsdurchlass derart mit dem zweiten Schnurdurchlass verbunden ist, dass die zweite Verstellschnur dazu ausgelegt ist, sich zwischen dem zweiten Schnurdurchlass und dem zweiten Schnurverriegelungsdurchlass zu bewegen, und
wobei die zweite Verstellschnur zwischen mindestens Folgendem verstellt werden kann:
einer ersten Position, die zu einer ersten Länge des Bandkörpers führt, und
einer zweiten Position, die zu einer zweiten Länge des Bandkörpers führt, wobei die zweite Länge kürzer als die erste Länge ist.

2. Verstellbares Brillenband nach Anspruch 1, wobei die Schnurverriegelung Folgendes umfasst:
einen Schnurdurchlass, der dazu ausgelegt ist, der Verstellschnur zu ermöglichen, durch die Schnurverriegelung zu verlaufen; und
einen Schnurverriegelungsdurchlass, der dazu ausgelegt ist, die Verstellschnur lösbar in Eingriff zu nehmen, um eine Bewegung der Verstellschnur durch die Schnurverriegelung zu verhindern,
wobei der Schnurverriegelungsdurchlass derart mit dem Schnurdurchlass verbunden ist, dass die Verstellschnur dazu ausgelegt ist, sich zwischen dem Schnurdurchlass und dem Schnurverriegelungsdurchlass zu bewegen, und
wobei die Verstellschnüre ein elastisches Material umfassen, sodass die Verstellschnur zu einer ursprünglichen Länge zurückkehrt, nachdem sie über die ursprüngliche Länge gedehnt wurde.

3. Verstellbares Brillenband nach Anspruch 1, wobei die Schnurverriegelung dazu ausgelegt ist, die Verstellschnur zu drehen, wenn sie durch die Schnurverriegelung verläuft, sodass ein Austrittsweg der Verstellschnur nicht parallel zu einem Eintrittsweg der Verstellschnur vor Eintritt der Verstellschnur in die Schnurverriegelung ist.

4. Verstellbares Brillenband nach Anspruch 3, wobei der Austrittsweg senkrecht zu dem Eintrittsweg ist.

5. Verstellbares Brillenband nach Anspruch 1,
wobei ein erster Abschnitt der Verstellschnur derart an einem ersten Ende der Verstellschnur mit dem Bandkörper verbunden ist, dass das erste Ende zu keiner Bewegung relativ zu dem Bandkörper in der Lage ist, und
wobei ein zweiter Abschnitt der Verstellschnur, der zwischen dem ersten Abschnitt und der Schnurverriegelung angeordnet ist, nicht an dem Bandkörper befestigt ist, sodass die Länge des zweiten Abschnitts variiert, wenn die Verstellschnur durch die Schnurverriegelung verstellt wird.

6. Verstellbares Brillenband nach Anspruch 1, wobei die Schnurverriegelung an dem Bandkörper angebracht ist.

7. Verstellbares Brillenband nach Anspruch 1, wobei die Schnurverriegelung dazu ausgelegt ist, die Verstellschnur von einer Innenseite des Bandkörpers zu einer Außenseite des Bandkörpers verlaufen zu lassen.

8. Verstellbares Brillenband nach Anspruch 7, wobei ein Endabschnitt der Verstellschnur, der an der Außenseite (18) angeordnet ist, ein Greifelement umfasst, das dazu ausgelegt ist, durch einen Benutzer gegriffen zu werden.

9. Verstellbares Brillenband nach Anspruch 1, ferner umfassend:
eine Haltevorrichtung (90), die an einem ersten Ende der Verstellschnur positioniert ist; und
eine Aufnahmevorrichtung, die an dem Bandkörper angebracht ist,
wobei die Haltevorrichtung dazu ausgelegt ist, die Aufnahmevorrichtung lösbar anzubringen.

10. Brillenanordnung, umfassend:
eine Brille (1); und
das verstellbare Brillenband nach Anspruch 1.

11. Brillenanordnung nach Anspruch 10,
wobei die Brille eine Linse (8) umfasst und
die Schnurverriegelung an der Linse positioniert ist.

12. Brillenanordnung nach Anspruch 10, wobei ein erstes Ende des Brillenbands an einem ersten Abschnitt der Brille angebracht ist und ein zweites Ende des Brillenbands an einem zweiten Abschnitt der Brille angebracht ist, sodass die Brille und das Brillenband eine Schleife bilden, die dazu ausgelegt ist, einen Kopf eines Benutzers zu umschließen.

13. Verstellbares Brillenband nach Anspruch 1, wobei die Verstellschnur eine Skala (49) umfasst, die dazu ausgelegt ist, eine Länge der Verstellschnur anzugeben.

14. Brillenanordnung nach Anspruch 10, ferner umfassend einen Brillenrahmen (4),
wobei die Schnurverriegelung einstückig mit dem Brillenrahmen ist.

15. Verstellbares Brillenband nach Anspruch 1, wobei eine Länge der Verstellschnur verstellt wird, indem die Schnur in einer Richtung senkrecht zu einer Ebene (79) gezogen wird, die tangential zu einer Außenfläche der Schnurverriegelung ist, und
wobei die Verstellschnur in der Schnurverriegelung gesichert wird, indem die Schnur in einer Richtung parallel zu der Außenebene gezogen wird.

16. Verstellbares Brillenband nach Anspruch 1, wobei:
die erste Position einem Zustand mit niedriger Spannung in dem verstellbaren Band entspricht und
die zweite Position einem Zustand mit hoher Spannung in dem verstellbaren Band entspricht.

## Revendications

1. Sangle de lunettes réglable (2), comprenant :
un corps de sangle (10) ayant une première extrémité (12) configurée pour la fixation à une première portion (6) de lunettes et une seconde extrémité (14) configurée pour la fixation à une seconde portion (7) des lunettes ;
un cordon de réglage (40) raccordé au corps de sangle ; et
un bloque-cordon (70) configuré pour la mise en prise de manière libérable du cordon de réglage à mesure qu'il passe à travers le bloque-cordon,
dans laquelle une portion du cordon de réglage située avant que le cordon de réglage n'entre dans le bloque-cordon est sensiblement parallèle au corps de sangle,
dans laquelle le cordon de réglage peut être réglé entre au moins :
une première position qui se traduit par une première longueur du corps de sangle, et
une seconde position qui se traduit par une seconde longueur du corps de sangle, dans laquelle la seconde longueur est inférieure à la première longueur,
un second cordon de réglage (44) raccordé au corps de sangle ; et
un second bloque-cordon configuré pour la mise en prise de manière libérable du second cordon de réglage à mesure qu'il passe à travers le second bloque-cordon, comprenant :
un second passage de cordon (72, 76) configuré pour la permission au second cordon de réglage de passer à travers le second bloque-cordon ; et
un second passage de blocage de cordon (74, 78) configuré pour la mise en prise de manière libérable du cordon de réglage pour la prévention d'un mouvement du cordon de réglage à travers le second bloque-cordon,
dans laquelle le second passage de blocage de cordon est interconnecté avec le second passage de cordon de telle sorte que le second cordon de réglage est configuré pour le déplacement entre le second passage de cordon et le second passage de blocage de cordon, et
dans laquelle le second cordon de réglage peut être réglé entre au moins :
une première position qui se traduit par une première longueur du corps de sangle, et
une seconde position qui se traduit par une seconde longueur du corps de sangle, dans laquelle la seconde longueur est inférieure à la première longueur.

2. Sangle de lunettes réglable selon la revendication 1, dans laquelle le bloque-cordon comprend :
un passage de cordon configuré pour la permission au cordon de réglage de passer à travers le bloque-cordon ; et
un passage de blocage de cordon configuré pour la mise en prise de manière libérable du cordon de réglage pour la prévention d'un mouvement du cordon de réglage à travers le bloque-cordon,
dans laquelle le passage de blocage de cordon est interconnecté avec le passage de cordon de telle sorte que le cordon de réglage est configuré pour le déplacement entre le passage de cordon et le passage de blocage de cordon, et
dans laquelle les cordons de réglage comprennent un matériau élastique de telle sorte que le cordon de réglage retourne à une longueur initiale après l'étirement au-delà de la longueur initiale.

3. Sangle de lunettes réglable selon la revendication 1, dans laquelle le bloque-cordon est configuré pour la rotation du cordon de réglage à mesure qu'il passe à travers le bloque-cordon de telle sorte qu'un trajet de sortie de cordon de réglage n'est pas parallèle à un trajet d'entrée du cordon de réglage avant l'entrée du cordon de réglage à l'intérieur du bloque-cordon.

4. Sangle de lunettes réglable selon la revendication 3, dans laquelle le trajet de sortie est perpendiculaire au trajet d'entrée.

5. Sangle de lunettes réglable selon la revendication 1,
dans laquelle une première portion du cordon de réglage est raccordée au corps de sangle au niveau d'une première extrémité du cordon de réglage de telle sorte que la première extrémité n'est pas capable d'un mouvement par rapport au corps de sangle, et
dans laquelle une seconde portion du cordon de réglage disposée entre la première portion et le bloque-cordon n'est pas fixée au corps de sangle, de telle sorte que la longueur de la seconde portion varie à mesure que le cordon de réglage est réglé à travers le bloque-cordon.

6. Sangle de lunettes réglable selon la revendication 1, dans laquelle le bloque-cordon est fixé au corps de sangle.

7. Sangle de lunettes réglable selon la revendication 1, dans laquelle le bloque-cordon est configuré pour le passage du cordon de réglage à partir d'un côté intérieur du corps de sangle vers un côté extérieur du corps de sangle.

8. Sangle de lunettes réglable selon la revendication 7, dans laquelle une portion d'extrémité du cordon de réglage qui est disposée sur le côté extérieur (18) comprend un élément de préhension configuré pour la préhension par un utilisateur.

9. Sangle de lunettes réglable selon la revendication 1, comprenant en outre :
un dispositif de retenue (90) positionné au niveau d'une première extrémité du cordon de réglage ; et
un récepteur fixé au corps de sangle,
dans laquelle le dispositif de retenue est configuré pour la fixation de manière libérable au récepteur.

10. Ensemble lunettes, comprenant :
des lunettes (1) ; et
la sangle de lunettes réglable selon la revendication 1.

11. Ensemble lunettes selon la revendication 10,
dans lequel les lunettes comprennent un verre (8), et
le bloque-cordon est positionné sur le verre.

12. Ensemble lunettes selon la revendication 10, dans lequel une première extrémité de la sangle de lunettes est fixée à une première portion des lunettes et une seconde extrémité de la sangle de lunettes est fixée à une seconde portion des lunettes de telle sorte que les lunettes et la sangle de lunettes forment une boucle configurée pour l'encerclement d'une tête d'un utilisateur.

13. Sangle de lunettes réglable selon la revendication 1, dans laquelle le cordon de réglage comprend une échelle (49) configurée pour l'indication d'une longueur du cordon de réglage.

14. Ensemble lunettes selon la revendication 10, comprenant en outre une monture de lunettes (4),
dans lequel le bloque-cordon est solidaire de la monture de lunettes.

15. Sangle de lunettes réglable selon la revendication 1, dans laquelle une longueur du cordon de réglage est réglée par la traction du cordon dans une direction perpendiculaire à un plan (79) tangent à une surface extérieure du bloque-cordon, et
dans laquelle le cordon de réglage est assujetti dans le bloque-cordon par la traction du cordon dans une direction parallèle au plan extérieur.

16. Sangle de lunettes réglable selon la revendication 1, dans laquelle :
la première position correspond à un état de basse tension dans la sangle réglable, et
la seconde position correspond à un état de haute tension dans la sangle réglable.
